# EUROPEAN PATENT APPLICATION

(11) **EP 2 311 883 A1**
(43) Date of publication of application: **20.04.2011**
(21) Application number: 09805091.7
(22) Date of filing: 05.08.2009
(51) Int. Cl.: C08B 15/05, A61K 47/36, A61L 31/00, C08B 37/08, C08K 3/00, C08L 1/08, C08L 5/00

(54) **HYDROGEL**

(30) Priority: 05.08.2008 JP 2008201906
(71) Applicant: Teijin Limited, Osaka-shi, Osaka 541-0054 (JP)
(72) Inventor: KANEKO, Hiroaki, Hino-shi Tokyo 191-0065 (JP); ITO, Masaya, Hino-shi Tokyo 191-0065 (JP); ENDO, Nobuyuki, Hino-shi Tokyo 191-0065 (JP); TANAKA, Taishi, Hino-shi Tokyo 191-0065 (JP)
(74) Representative: Hallybone, Huw George
(86) International application number: PCT/JP2009/064211
(87) International publication number: WO 2010/016611

(57) **Abstract**

A hydrogel comprising a polysaccharide, wherein amphiphilic side chains are bonded to the carboxyl groups of a polysaccharide with carboxyl groups on side chains, an inorganic ion other than calcium ion, and water, and a method for its preparation. The gel has high viscoelasticity, can be injected into the body using an instrument such as a syringe, and is useful for various biomedical materials.

## Description

### Technical Field

The present invention relates to a gel comprising a polysaccharide, wherein amphiphilic side chains are bonded to the carboxyl groups of a polysaccharide having carboxyl groups on side chains, and an inorganic salt aqueous solution. It is useful as a medical material because of its excellent flow property and viscoelasticity.

### Background Art

Introduction of various functional groups into the side chains of polysaccharides is known to modify their physical properties and shapes, sometimes improving the physical properties of the polysaccharides themselves or the flow properties of the polysaccharide aqueous solutions, or forming hydrogels, and a variety of polysaccharide derivatives and their modifying methods have been reported. Polysaccharides also exist that are polysaccharides with carboxyl groups on side chains, with phospholipids bonded thereto.

Japanese Unexamined Patent Application Publication No. 2006-296916 describes an adhesion prevention material comprising a reaction product of hyaluronic acid and phosphatidylethanolamine. Also, WO2007/015579 describes an adhesion prevention material comprising a reaction product of carboxymethylcellulose and phosphatidylethanolamine.

However, neither of these publications mention or suggest the hydrogel disclosed in the present specification.

Polysaccharides that gel with inorganic ions are known, but these are only of such types that gel with the divalent cation calcium ion, such as sodium alginate or pectin aqueous solutions. While polysaccharides that gel with calcium ion do indeed exhibit excellent gel-forming ability, the fact that calcium ion is also a factor governing neural transmission, and performing numerous physiological roles as well including activation of blood clotting factors, has limited their uses in medical gels.

On the other hand, polysaccharides that gel with monovalent cations such as sodium ion are unknown.

### SUMMARY OF THE INVENTION

### Problems to be Solved by the Invention

An object of the present invention is to provide a hydrogel that, without using calcium ion, has a complex elastic modulus suitable for use in the body and also has excellent safety and usability.

### Means for Solving the Problems

As a result of much diligent research directed toward this object, the present inventors have completed this invention upon finding that a specific type of polysaccharide derivative can gel without using calcium ion.

Specifically, the invention is a hydrogel comprising a polysaccharide, wherein amphiphilic side chains are bonded to the carboxyl groups of a polysaccharide with carboxyl groups on side chains (hereinafter also referred to as "component 1"), an inorganic ion other than calcium ion (hereinafter also referred to as "component 2"), and water.

The invention is also a kit for preparation of a hydrogel, which is constructed from two solutions: an aqueous solution of component 1 and an aqueous solution of component 2.

The invention is also a method for preparing a hydrogel, which comprises a step of mixing an aqueous solution of component 1 and an aqueous solution of component 2.

The invention is also a method for preparing a hydrogel, which comprises a step of dissolving component 1 in an aqueous solution of component 2.

### Effect of the Invention

The hydrogel of the invention is a highly viscous gel, and it is preferably used as biomedical materials, which includes the use as an injectable gel that can be injected with a syringe, for example. The hydrogel of the invention is suitable for use in the body, since it gels without the use of calcium.

### Best Mode for Carrying Out the Invention

Component 1 in the hydrogel of the invention is a polysaccharide wherein amphiphilic side chains are bonded to the carboxyl groups of a polysaccharide having carboxyl groups on side chains.

Polysaccharides having carboxyl groups on side chains include polysaccharides having on the main chain monosaccharides with carboxyl groups such as alginic acid, hyaluronic acid or pectin, carboxyalkylated polysaccharides such as carboxymethyl cellulose, carboxymethyl chitin, carboxymethyl pullulan, carboxymethyl dextran, carboxymethyl starch, carboxyethyl cellulose, carboxymethyl chitosan, and reaction products of cyclic carboxylic anhydrides and polysaccharides such as N-succinylchitosan, obtained by reaction between chitosan and succinic anhydride. The carboxyl groups of these polysaccharides may form salts with alkali metals such as sodium, potassium or lithium.

In the case of a carboxyalkylated polysaccharide, there are no particular restrictions on the degree of substitution or locations of substitution of the carboxyalkyl groups, but preferably the derivative has a degree of substitution of 0.6-1.0 and the locations of substitution are the C-6 primary hydroxyl groups.

The molecular weight of the polysaccharide is not particularly restricted, but it is preferably 10,000-2,000,000, more preferably 20,000-1,500,000 and even more preferably 30,000-1,000,000, in terms of weight-average molecular weight.

Of the aforementioned polysaccharides it is preferred to use carboxymethyl cellulose or hyaluronic acid, because these readily gel when mixed with inorganic salts, and especially sodium salts.

The amphiphilic side chains that bond to the carboxyl groups are not particularly restricted so long as they are functional groups having both a hydrophilic functional group and a hydrophobic functional group, and preferred examples thereof include side chains with a phosphatidylethanolamine structure, as a phospholipid type. Particularly preferred are those wherein the carboxyl group of a polysaccharide and the primary amino group of a phosphatidylethanolamine are directly bonded by an amide bond.

Specific phosphatidylethanolamine compounds include dilauroylphosphatidylethanolamine, dimyristoylphosphatidylethanolamine, dipalmitoylphosphatidylethanolamine, distearoylphosphatidylethanolamine, diarachidoylphosphatidylethanolamine, dibehenoylphosphatidylethanolamine, laurooleoylphosphatidylethanolamine, myristoleoylphosphatidylethanolamine, palmitoleoylphosphatidylethanolamine, dioleoylphosphatidylethanolamine, dilinoleoylphosphatidylethanolamine, dilinolenoylphosphatidylethanolamine, diarachidonoylphosphatidylethanolamine, didocosahexaenoylphosphatidylethanolamine and the like, with phosphatidylethanolaminedioleoyl being preferred among these.

Other preferred amphiphilic side chains include straight-chain types with the following structural formula.

### (Structural formula) Polysaccharide-CO-NH-X-CO-Y-Z

Here, CO is from the polysaccharide carboxyl group, X is a C1-10 divalent hydrocarbon group,
Y is a divalent polyalkylene oxide having oxygen atoms at both ends, and
Z is a C1-24 hydrocarbon group or -CO-R¹ (R¹ being a C1-23 hydrocarbon group).

X in the formula is a C1-10 divalent hydrocarbon group, and specifically it may be a methylene, ethylene, n-propylene, isopropylene, n-butylene or isobutylene group. It is preferably a methylene group, in which case it is a bonding unit using glycine, as it will be adjacent to a carbonyl group and an amino group.

Y is a divalent polyalkylene oxide having oxygen atoms at both ends. A divalent polyalkylene oxide is, specifically, a polyalkylene ether such as polyethylene glycol, polypropylene glycol or polybutylene glycol. By "having oxygen atoms at both ends" is meant a structure in which the hydroxyl groups at both ends of the polyalkylene ether contribute to bonding with the adjacent functional groups. Specifically, there may be mentioned 1,2-polypropyleneglycols represented by -(O-CH₂-CH₂(CH₃)-)ₙ-O-, 1,3-polypropyleneglycols represented by -(O-CH₂-CH₂-CH₂-)ₙ-O-, and polyethylene glycols represented by -O-CH₂-CH₂-)ₙ-O-. It may also be a copolymer of polyethylene glycol and polypropylene glycol, such as a copolymer represented by PEO-PPO, for example. Here, n represents the number of repeating units.

Preferred among these are structures having polyethylene glycol as the main repeating unit, and specifically those comprising at least 80 mol% and more preferably at least 90 mol% polyethylene glycol units. The number of repeating units n is preferably 2-100 and more preferably 3-70.

Z is a C1-24 hydrocarbon group or -CO-R¹ (R¹ being a C1-23 hydrocarbon group).

Specific examples of C1-24 hydrocarbon groups for Z include straight-chain alkyl groups such as methyl, ethyl, propyl, butyl, pentyl, hexyl, octyl, nonyl, lauryl and stearyl, alkyl groups with cyclic structures such as cyclohexyl, cyclopentyl, cyclohexylnonyl and cholesteryl, unsaturated alkyl groups such as oleyl, and aromatic hydrocarbon groups such as phenyl, naphthyl and benzyl. Stearyl and oleyl groups are preferred among these.

R¹ is a C1-23 hydrocarbon group.

Specific examples for R¹ include straight-chain alkyl groups such as methyl, ethyl, propyl, butyl, pentyl, hexyl, octyl, nonyl, heptadecanyl, heptadecenyl, lauryl and stearyl, alkyl groups with cyclic structures such as cyclohexyl, cyclopentyl, cyclohexylnonyl and cholesteryl, unsaturated alkyl groups such as oleyl, and aromatic hydrocarbon groups such as phenyl, naphthyl and benzyl. Heptadecanyl and heptadecenyl groups are preferred among these.

When R¹ is an aliphatic alkyl group, -CO-R¹ will be an acyl group derived from a fatty acid. As specific preferred examples there may be mentioned lauroyl, palmitoyl, stearoyl and oleoyl. When R¹ is an aromatic group, -CO-R¹ will be an acyl group derived from an aromatic fatty acid, and benzoyl and naphthoyl may be mentioned as specific examples. Stearoyl and oleoyl groups are preferred among these.
Preferred examples of amphiphilic side chains represented by -NH-X-CO-Y-Z include the following.
Side chain compound groups having polyethylene glycol in the hydrophilic portion and an alkyl group in the hydrophobic portion, such as -NH-CH₂-CO-(OCH₂CH₂)7-O-C₁₈H₃₇ and -NH-CH₂CH₂-CO- (OCH₂CH₂)₇-O-C₁₈H₃₇;
Side chain compound groups having polyethylene glycol in the hydrophilic portion and an alkenyl group in the hydrophobic portion, such as -NH-CH₂-CO-(OCH₂CH₂)₇-O-C₁₈H₃₅ and -NH-CH₂-CO-(OCH₂CH₂)₂₀-O-C₁₈H₃₅;
Side chain compound groups having polyethylene glycol in the hydrophilic portion and a straight-chain fatty acid ester in the hydrophobic portion, such as -NH-CH₂-CO-(OCH₂CH₂)₂₀-O-CO-C₁₇H₃₅;
Side chain compound groups having polyethylene glycol in the hydrophilic portion and a cholesteryl group in the hydrophobic portion, such as -NH-CH₂-CO-(OCH₂CH₂)₇-O-cholesteryl.

Bonding between the carboxyl groups and the amphiphilic side chains of the polysaccharide may be accomplished by, for example, bonding using a condensation agent such as a carbodiimide. As carbodiimides there may be mentioned 1-ethyl-3-(dimethylaminopropyl)-carbodiimide and its hydrochloride, diisopropylcarbodiimide, dicyclohexylcarbodiimide and N-hydroxy-5-norbornane-2,3-dicarboximide. Preferred for use among these is 1-ethyl-3-(dimethylaminopropyl)-carbodiimide hydrochloride.

A carboxyl activating reagent is preferably used for activation of the polysaccharide carboxyl groups. As carboxyl activating reagents there may be mentioned N-hydroxysuccinimide, p-nitrophenol, N-hydroxybenzotriazole, N-hydroxypiperidine, N-hydroxysuccinamide, 2,4,5-trichlorophenol and N,N-dimethylaminopyridine. N-hydroxybenzotriazole is preferred among these.

The reaction solvent may be water alone, or a mixture of water and a compatible organic solvent may be used, and reaction may even be conducted in a two layer system employing an organic solvent that is not compatible with water. As organic solvents that are compatible with water there may be mentioned alcohols such as methanol and ethanol, cyclic ethers such as tetrahydrofuran and dioxane, ethers such as polyethylene oxide compounds, amides such as dimethylformamide and dimethylacetamide, amines such as pyridine and piperidine, dialkylsulfones such as dimethyl sulfoxide and ketones such as acetone. Preferably, carboxymethylcellulose and a phospholipid are reacted in a homogeneous reaction system comprising a mixture of water and an organic solvent that is compatible with water. The organic solvent that is compatible with water is preferably tetrahydrofuran.

The temperature for the reaction using the carbodiimide is preferably 0-60°C. The reaction is more preferably conducted at 0-10°C to inhibit by-products. The reaction environment is preferably weakly acidic and even more preferably pH 6-7.

When the amphiphilic side chains are phospholipids, the degree of substitution is preferably 0.3-2.0 mol%/sugar residue. A lower degree of substitution will not allow sufficient viscoelasticity to be obtained as a gel, and a higher one will tend to form a hard gel that is insoluble in water. The degree of substitution is preferably 0.6-1.9, and even more preferably 0.7-1.7 mol%/sugar residue.

Naturally, this preferred degree of substitution will vary depending on the type and molecular weight of polysaccharide used, the degree of substitution and locations of substitution of the carboxyl groups, the types of amphiphilic side chains, the composition or concentration of inorganic ions other than calcium ion, and the final concentration of the hydrogel. However, if a person skilled in the art carefully considers that an excessively high degree of substitution will tend to form an insoluble, hard gel, the preferred ranges for the degree of substitution may be easily determined under individual conditions under testing, with reference to the concrete examples provided in the Examples described below.

When preparing an aqueous solution of component 1, preferably 0.1-3.0 parts by weight, more preferably 0.3-2.0 parts by weight and even more preferably 0.5-1.0 part by weight of component 1 is used with respect to 100 parts by weight of water.

Other components that might be included in component 1 are the condensation agent used for synthesis, by-products such as urea generated by the condensation agent undergoing certain chemical reactions, the carboxyl activating reagent, unreacted amines, contaminants that may become included at different stages of the reaction and ions used to adjust the pH, and these components are preferably limited to a low level such that none of the compounds produce noticeable contaminant reactions when the gel is placed in the body. Naturally, solutions including such components are encompassed within the scope of the invention.

When preparing an aqueous solution of component 1, the aqueous solution may contain other polysaccharides or water-soluble polymers, monosaccharides or oligosaccharides, amino acids, or the like, as desired. In addition, low molecular drugs or peptides and proteins having physiological activity may also be included as desired.

The inorganic salt of component 2 is a salt comprising both a cation component and an anion component, wherein the cation component and anion component are preferably present in equimolar amounts. As such cation components there may be mentioned ions of alkali metals such as sodium, lithium and potassium, or ammonium ion, and they may contain protons in some cases, as with sodium hydrogenphosphate. Salts with sodium ions, that are abundant in the body, are preferably used among these.

There are no particular restrictions on the anion component, and there may be mentioned halide ions such as chloride ion or bromide ion, ions such as phosphate ion and sulfate ion, and anions of carboxylic acids such as acetate, citrate or oxalate.

As specific examples of inorganic salts of component 2 there may be mentioned alkali metal salts such as sodium chloride, sodium fluoride, sodium bromide, potassium chloride, sodium acetate, monosodium hydrogenphosphate, disodium hydrogenphosphate, sodium phosphate, sodium sulfate, sodium citrate and sodium oxalate, as well as sodium tartrate, sodium potassium tartrate, sodium hydrogencarbonate, sodium carbonate, potassium carbonate, and the like.

Of these salts, sodium chloride is preferred from the viewpoint of safety. The sodium chloride concentration may be in the range of 0.1-10%, but it is more preferably 0.5-5% and even more preferably 1-3%.

When the main purpose is to support cell survival, the type and amount of ion is selected for adjustment to a physiological salt concentration. In this case, it is preferred to use the composition or concentration of the physiological saline (0.9% NaCl aqueous solution), Ringer's solution, Locke's solution or the like serving as the physiological salt solution.

The aqueous solution of component 2 also preferably contains a water-soluble polymer. The water-soluble polymer is preferably selected as one having an appropriate viscosity for ease of admixture during mixture with the solid component 1 or the viscoelastic aqueous solution of component 1, or for ease of use.

Specific examples of such water-soluble polymers include water-soluble polysaccharides such as sodium alginate, sodium hyaluronate, pectin, carrageenan, starch, dextrin, dextran, pullulan, heparin and chitosan, water-soluble polysaccharide derivatives such as carboxymethyl cellulose, carboxymethyl dextrin, carboxymethyl pullulan and carboxyethyl cellulose, proteins such as gelatin, collagen, albumin, fibrinogen, thrombin and fibroin, nucleic acids such as DNA or RNA, and synthetic polymers, including polyethylene glycol and its copolymers, polycations such as polyvinyl alcohol or its copolymers and polyallylamine or its copolymers, and polyanions such as sodium polyacrylate or its copolymers.

Particularly preferred for use among these, as medical materials, are sodium hyaluronate, carboxymethylcellulose sodium and polyethylene glycol or its copolymers.

An aqueous solution of component 2 may contain other monosaccharides or oligosaccharides, amino acids, or the like, as desired. In addition, low molecular drugs or peptides and proteins having physiological activity may also be included as desired.

The preferred complex elastic modulus for the hydrogel of the invention, when the amphiphilic side chains are phospholipids, is 20-1000 N/m², more preferably 25-500 N/m² and even more preferably 30-200 N/m², as measured under conditions with a polymer concentration of 1.0 wt% in water and a temperature of 37°C, using a dynamic viscoelasticity measuring apparatus, or "rheometer", at an angular velocity of 10 rad/sec. The complex elastic modulus is the constant representing the ratio of the stress and strain of the elastic body.

The hydrogel of the invention has significantly increased viscoelasticity by admixture of two components, which allows it to remain at a prescribed site in the body, and it is therefore potentially useful for protection of wounds and formation of physical isolation barriers between organs.

In addition, a local drug delivery system may be provided by impregnating the hydrogel of the invention with a drug.

Moreover, the property of decomposing or being absorbed upon injection into the body renders the hydrogel of the invention useful as an injection gel material or a scaffolding material for regenerative medicine.

The hydrogel of the invention may also be used for medical purposes including biomedical materials, for daily commodities such as hair care products or skin humectants, or for cosmetic uses.

The hydrogel of the invention also encompasses forms that are injectable through syringes, which may be used for low invasive medical care. It may also be used, in particular, as a cell carrier for regenerative medicine, as a carrier for retention or sustained release of liquid factors such as growth factors, as a carrier for retention or sustained release of low molecular compound drugs, or as a biomedical material such as an adhesion prevention material or sealant.

It may further be used as a cell culture carrier, microbial culture carrier or dental implant material.

The hydrogel of the invention may be subjected to sterilization treatment by a known sterilization method. Preferred sterilization methods are electron beam irradiation, gas sterilization with ethylene oxide, and high-pressure steam sterilization.

The suitable components for component 1 or component 2, mentioned above for the hydrogel of the invention, or their aqueous solutions, are likewise appropriate for a kit of the invention or the method of the invention.

The invention further encompasses a method for preparing a gel according to the invention. Specifically, it is a method of preparing a hydrogel comprising a step of mixing an aqueous solution of component 1 and an aqueous solution of component 2 (preparation method 1), or a method of preparing a hydrogel comprising a step of dissolving component 1 in an aqueous solution of component 2 (preparation method 2).

The aqueous solution of component 1 for preparation method 1 may contain components other than water-soluble polymers, as mentioned above, or it may be dissolved in water without containing an ion.

In preparation method 2, on the other hand, the solid component 1 is dissolved in an aqueous solution of component 2, and therefore its form is preferably selected as appropriate from the viewpoint of the dissolution rate. For example, component 1 may be dissolved after preparation as a powder.

### Examples

(1) The following materials were used in the examples.
   (i) CMCNa: Carboxymethylcellulose sodium (SEROGEN PM-250L, product of Dai-ichi Kogyo Seiyaku Co., Ltd., carboxymethyl group degree of substitution: 0.73),
   (ii) CMCNa: Carboxymethylcellulose sodium (P-603A, product of Dai-ichi Kogyo Seiyaku Co., Ltd., degree of substitution: 0.69),
   (iii) CMCNa: Carboxymethylcellulose sodium (product of Nippon Paper Chemicals Co., Ltd., degree of substitution: 0.69),
   (iv) HANa: Sodium hyaluronate (FCH-80LE, product of Kibun FoodChemifa Co., Ltd.),
   (v) Tetrahydrofuran (product of Wako Pure Chemical Industries, Ltd.),
   (vi) 0.1 M HCl (product of Wako Pure Chemical Industries, Ltd.),
   (vii) 0.1 M NaOH (product of Wako Pure Chemical Industries, Ltd.),
   (viii) EDC: 1-Ethyl-3-[3-(dimethylamino)propyl]-carbodiimide·HCl (product of Osaka Synthetic Chemical Laboratories, Inc.),
   (ix) HOBt·H₂O: 1-Hydroxybenzotriazole monohydrate (product of Osaka Synthetic Chemical Laboratories, Inc.),
   (x) DMT-MM: 4-(4,6-Dimethoxy-1,3,5-triazin-2-yl)-4-methylmorpholinium chloride (product of Kokusan Chemical Co., Ltd.),
   (xi) L-α-Dioleoylphosphatidylethanolamine (COATSOME ME-8181, product of NOF Corp.),
   (xii) Ethanol (product of Wako Pure Chemical Industries, Ltd.),
   (xiii) NaCl (product of Wako Pure Chemical Industries, Ltd.),
   (xiv) KCl (product of Wako Pure Chemical Industries, Ltd.),
   (xv) Distilled water for injection (product of Otsuka Pharmaceutical Co., Ltd.).
(2) Measurement of phospholipid content in cellulose derivative

The proportion of phospholipids in the cellulose derivative was determined by analyzing the total phosphorus content by vanadomolybdate absorptiometry.

### (3) Measurement of complex elastic modulus of hydrogel

The complex elastic modulus of the hydrogel was measured at 37°C with an angular velocity of 10 rad/sec, using a Rheometer RFIII (TA Instrument) as the dynamic viscoelasticity measuring apparatus. The complex elastic modulus is the constant representing the ratio of the stress and strain of the elastic body.

### Example 1

After dissolving 1500 mg of CMCNa (PM-250L, product of Dai-ichi Kogyo Seiyaku Co., Ltd., degree of substitution: 0.73) in 300 mL of water, 300 mL of tetrahydrofuran was further added. To this solution there were added 709.7 mg of L-α-dioleoylphosphatidylethanolamine and 290.4 mg of DMT-MM as a condensation agent, and then the mixture was stirred overnight. After the stirring, the mixture was added to ethanol for precipitation. The ethanol was removed by filtration, washing was performed again with ethanol, and the filtrate was vacuum dried to obtain a cellulose derivative, after which the phospholipid content was measured. The phospholipid content was used for calculation to determine the degree of substitution of phosphatidylethanolamine, and a value of 0.71 mol%/sugar residue was obtained.

After dissolving 20 mg of the obtained cellulose derivative in 1800 mg of distilled water for injection, 200 mg of 9% NaCl was added to a final concentration of 0.9%, to prepare a hydrogel with a final concentration of 1.0 wt%. The obtained hydrogel did not flow even when the container was inclined. The complex elastic modulus of the hydrogel was measured to be 249.1 ±28.3 N/m² (mean ±SD).

### Comparative Example 1

A hydrogel was prepared by the same procedure as Example 1, except that 200 mg of distilled water for injection was added instead of 9% NaCl. The obtained hydrogel flowed slowly when the container was inclined, and the liquid surface exhibited a tendency to move horizontally. The complex elastic modulus of the hydrogel was measured to be 36.6 ±0.43 N/m² (mean ±SD).

### Example 2

A hydrogel was prepared by the same procedure as Example 1, except that after dissolving 40 mg of cellulose derivative in 1800 mg of distilled water for injection, 200 mg of 9% NaCl was added to a final concentration of 0.9%, and the final concentration was 2.0 wt%. The complex elastic modulus of the obtained hydrogel was measured to be 955.9 ±32.0 N/m² (mean ±SD).

### Example 3

A cellulose derivative was obtained by the same method as Example 1, except that there were added to the reaction system 1421.0 mg of L-α-dioleoylphosphatidylethanolamine and 581.4 mg of DMT-MM, as a condensation agent, and the phospholipid content was measured. The degree of substitution was 2.2 mol%/sugar residue.

After dissolving 20 mg of cellulose derivative in 1800 mg of distilled water for injection, 200 mg of 9% NaCl was added to a final concentration of 0.9%, to prepare a hydrogel with a final concentration of 1.0 wt%. As a result, a somewhat opaque, hard hydrogel was obtained that was insoluble even in water. The precise value of its complex elastic modulus could not be measured because the gel was hard and because of significant slipping against the rheometer measuring surface.

Thus it was confirmed that, with a hydrogel prepared from a cellulose derivative having a degree of substitution of approximately 0.75 mol%/sugar residue, addition of NaCl to 0.9 wt%, approximating the concentration in the body, resulted in a notable increase in the complex elastic modulus. On the other hand, with a hydrogel prepared from a cellulose derivative having a degree of substitution of approximately 2.2 mol%/sugar residue, addition of NaCl to 0.9 wt% was able to yield a hard gel that was insoluble even in water. These results demonstrated that a hydrogel had been obtained having a property of hardening at a sodium chloride concentration approximating that in the body.

### Example 4

After dissolving 3000 mg of CMCNa (F600MC, product of Nippon Paper Chemicals Co., Ltd., degree of substitution: 0.69) in 600 mL of water, 600 mL of tetrahydrofuran was added. Synthesis was carried out in the same manner as Example 1, except that 2839 mg of L-α-dioleoylphosphatidylethanolamine, 807 mg of EDC as a condensation agent and 643 mg of HOBt·H₂O were added to the solution, and the mixture was stirred overnight. The degree of substitution of phosphatidylethanolamine in the obtained polysaccharide derivative was 0.8 mol%/sugar residue.

After dissolving 20 mg of the obtained cellulose derivative in 1800 mg of distilled water for injection, 200 mg of 9% NaCl was added to a final concentration of 0.9%, to prepare a hydrogel with a final concentration of 1.0 wt%. The obtained hydrogel did not flow even when the container was inclined, and the complex elastic modulus was 313.4 N/m².

### Comparative Example 2

A hydrogel was prepared by the same procedure as Example 4, except that 200 mg of distilled water for injection was added instead of 9% NaCl. The complex elastic modulus of the obtained hydrogel was 121.3 N/m².

### Example 5

A hydrogel was prepared in the same manner as Example 4 using the polysaccharide derivative obtained in Example 4, except that CMCNa (F30MC, product of Nippon Paper Chemicals Co., Ltd.) was present at 1.0 wt% in the 9% NaCl aqueous solution. The complex elastic modulus of the obtained hydrogel was 343.0 N/m².

### Example 6

A hydrogel was prepared in the same manner as Example 4 using the polysaccharide derivative obtained in Example 4, except that polyethylene glycol (product of Wako Pure Chemical Industries, Ltd., molecular weight: 20,000) was present at 3% in the 9% NaCl aqueous solution. The complex elastic modulus of the obtained hydrogel was 393.6 N/m².

### Example 7

A polysaccharide derivative was synthesized in the same manner as Example 1, except that F30MC by Nippon Paper Chemicals Co., Ltd. (degree of substitution of carboxymethyl groups: 0.69) was used as the carboxymethylcellulose. The degree of substitution of phosphatidylethanolamine in the obtained polysaccharide derivative was 0.8 mol%/sugar residue.

After dissolving 20 mg of this polysaccharide derivative in 1800 mg of distilled water for injection, 200 mg of 9% NaCl was added to a final concentration of 0.9%, to prepare a hydrogel with a final concentration of 1.0 wt%. The complex elastic modulus of the obtained hydrogel was 181 N/m².

### Example 8

A hydrogel was prepared in the same manner as Example 7, except for using a 9% KCl aqueous solution. The complex elastic modulus of the obtained hydrogel was 180 N/m².

### Comparative Example 3

A hydrogel was prepared by the same procedure as Example 7, except that 200 mg of distilled water for injection was added instead of 9% NaCl. The complex elastic modulus of the obtained hydrogel was measured to be 15.3 N/m².

### Example 9

After dissolving 1500 mg of sodium hyaluronate (FCH-80LE, product of Kibun FoodChemifa Co., Ltd.) in 300 mL of water, 300 ml of tetrahydrofuran was further added. To this solution there was added a solution of 1113 mg (0.01496 mol) of L-α-dioleoylphosphatidylethanolamine (40 equivalents to 100 equivalents of carboxyl groups of HA-Na), 316 mg (0.01645 mol) of EDC and 251.8 mg (0.016456 mol) of HOBt·H₂O in 75 mL of tetrahydrofuran/water = 1/1, and the mixture was stirred overnight. After stirring, the tetrahydrofuran was removed and the mixture was added to ethanol for precipitation after some evaporation of the water. The ethanol was removed by filtration and washed again with ethanol, and the filtrate was vacuum dried to obtain a hyaluronic acid derivative.

After dissolving 20 mg of the obtained hyaluronic acid derivative in 1800 mg of distilled water for injection, 200 mg of 9% NaCl was added to a final concentration of 0.9%, to prepare a hydrogel with a final concentration of 1.0 wt%. The obtained hydrogel did not flow even when the container was inclined. The complex elastic modulus of the hydrogel was measured to be 139.4 ±16.4 N/m² (mean ±SD).

### Comparative Example 4

A hydrogel was prepared by the same procedure as Example 9, except that 200 mg of distilled water for injection was added instead of 9% NaCl. The complex elastic modulus of the obtained hydrogel was measured to be 56.9 ±9.1 N/m² (mean ±SD).

### Example 10

### Synthesis of H₂N-CH₂-CO-(O-CH₂CH₂)₇-O-C₁₈H₃₅

After dissolving 1 millimole of N-butyloxycarbonylglycine (Boc-Gly-OH, product of Wako Pure Chemical Industries, Ltd.) with respect to 1 millimole of oleyl alcohol polyethyleneglycol ether (H-(O-CH₂CH₂)₇-O-C₁₈H₃₅, product of Wako Pure Chemical Industries, Ltd.) in dichloromethane, a dichloromethane solution containing 1 millimole of dicyclohexylcarbodiimide (product of Wako Pure Chemical Industries, Ltd.) as a condensation agent was added dropwise at room temperature. The reaction mixture was filtered to remove the dicyclohexylurea by-product and then concentrated and dried to obtain an amino group-protected intermediate (Boc-NH-CH₂-CO-(O-CH₂CH₂)₇-O-C₁₈H₃₅).

Approximately 1-2 mL of trifluoroacetic acid (product of Wako Pure Chemical Industries, Ltd.) was added to the intermediate, and de-Boc reaction by acid treatment was conducted at room temperature for 2 hours. Progress of the reaction was confirmed by TLC. The reaction mixture was concentrated under reduced pressure, and the excess trifluoroacetic acid was removed to obtain a trifluoroacetic acid salt of an amine compound as the target product. The product was confirmed by ¹H-NMR.

### Example 11

### Coupling of carboxymethylcellulose (CMC-Na) and H₂N-CH₂-CO-(O-CH₂CH₂)₇-O-C₁₈H₃₅

After dissolving 1500 mg of CMC-Na (F600MC, product of Nippon Paper Chemicals Co., Ltd., degree of substitution: 0.69) in 300 ml of water, 300 ml of tetrahydrofuran was further added and mixed therewith to obtain a homogeneous solution. The trifluoroacetate of H₂N-CH₂-CO-(O-CH₂CH₂)₇-O-C₁₈H₃₅ synthesized in Example 10 was added and mixed at 0.2 equivalent to 1 equivalent of carboxyl groups in the CMC-Na.

DMT-MM, as a condensation agent, was dissolved in 30 ml of tetrahydrofuran/water = 1/1 at 1.1 equivalents with respect to the H₂N-CH₂-CO-(O-CH₂CH₂)₇-O-C₁₈H₃₅, and after addition to the reaction system, it was stirred overnight. After stirring, the reaction mixture was concentrated with a rotary evaporator to remove the tetrahydrofuran, the water was evaporated off, and the total amount was concentrated to approximately 1/3, after which the reaction mixture was added to ethanol to form a precipitate. The precipitate was filtered and the resulting precipitate was suspended in ethanol and stirred for 24 hours, and then recovered and vacuum dried to obtain a cellulose derivative. The obtained cellulose derivative was subjected to elemental analysis and the degree of substitution was calculated from the proportion of carbon and nitrogen. As a result, the degree of substitution was 13 mol%/sugar residue.

### Example 12

After dissolving 20 mg of the derivative obtained in Example 11 in 1800 mg of distilled water for injection, 200 mg of 9% NaCl was added to a final concentration of 0.9%, to prepare a hydrogel with a final concentration of 1.0 wt%. The obtained hydrogel did not flow even when the container was inclined. The complex elastic modulus of the hydrogel was measured to be 1448.4 N/m².

### Comparative Example 5

A hydrogel was prepared by the same procedure as Example 12, except that 200 mg of distilled water for injection was added instead of 9% NaCl. The complex elastic modulus of the hydrogel was measured to be 729.7 N/m² .

### Example 13

After dissolving 3000 mg of CMCNa (P-603A, product of Dai-ichi Kogyo Seiyaku Co., Ltd., degree of substitution: 0.69) in 200 mL of water, 200 mL of tetrahydrofuran was further added. To this solution there were added 349 mg of L-α-dioleoylphosphatidylethanolamine and 646 mg of DMT-MM as a condensation agent, and then the mixture was stirred overnight. After the stirring, the mixture was added to ethanol for precipitation. The ethanol was removed by filtration, washing was performed again with ethanol, and the filtrate was vacuum dried to obtain a cellulose derivative, after which the phospholipid content was measured. The phospholipid content was used for calculation to determine the degree of substitution of phosphatidylethanolamine, and a value of 1.31 mol%/sugar residue was obtained.

After dissolving 30 mg of the obtained cellulose derivative in 2700 mg of distilled water for injection, 270 mg of a 10-fold concentration of PBS(-) aqueous solution (NaCl: 8%, NaH₂PO₄: 0.35%, Na₂HPO₄: 1.28%) was added for a final concentration of NaCl: 0.8%, NaH₂PO₄: 0.035%, Na₂HPO₄: 0.128%, to prepare a hydrogel with a final concentration of 1.0 wt%. The obtained hydrogel did not flow even when the container was inclined. The complex elastic modulus of the hydrogel was measured to be 101.6 ±3.4 N/m² (mean ±SD).

### Example 14

A 40 mg portion of cellulose derivative synthesized by the method described in Example 13 was dissolved in 3960 mg of PBS(-) aqueous solution (NaCl: 0.8%, NaH₂ PO₄ : 0.035%, Na₂HPO₄ : 0.128%), and a hydrogel with a final concentration of 1.0 wt% was prepared. The obtained hydrogel did not flow even when the container was inclined. The complex elastic modulus of the hydrogel was measured to be 70.9 ±1.5 N/m² (mean ±SD).

### Example 15

A 30 mg portion of cellulose derivative synthesized by the method described in Example 13 was dissolved in 2970 mg of a sodium hydrogenphosphate aqueous solution (0.17%), and a hydrogel with a final concentration of 1.0 wt% was prepared. The obtained hydrogel did not flow even when the container was inclined. The complex elastic modulus of the hydrogel was measured to be 16.9 ±0.4 N/m² (mean ±SD).

### Example 16

A 30 mg portion of cellulose derivative synthesized by the method described in Example 13 was dissolved in 2970 mg of a sodium hydrogenphosphate (0.17%) and sodium chloride (0.27%) aqueous solution, and a hydrogel with a final concentration of 1.0 wt% was prepared. The obtained hydrogel did not flow even when the container was inclined. The complex elastic modulus of the hydrogel was measured to be 72.7 ±4.1 N/m² (mean ±SD).

### Example 17

A 30 mg portion of cellulose derivative synthesized by the method described in Example 13 was dissolved in 2970 mg of a sodium hydrogenphosphate (0.17%) and sodium chloride (0.53%) aqueous solution, and a hydrogel with a final concentration of 1.0 wt% was prepared. The obtained hydrogel did not flow even when the container was inclined. The complex elastic modulus of the hydrogel was measured to be 91.5 ±0.5 N/m² (mean ±SD).

### Example 18

A 30 mg portion of cellulose derivative synthesized by the method described in Example 13 was dissolved in 2970 mg of a sodium hydrogenphosphate (0.17%) and sodium chloride (0.8%) aqueous solution, and a hydrogel with a final concentration of 1.0 wt% was prepared. The obtained hydrogel did not flow even when the container was inclined. The complex elastic modulus of the hydrogel was measured to be 87.6 ±3.1 N/m² (mean ±SD).

### Comparative Example 6

A 30 mg portion of cellulose derivative synthesized by the method described in Example 13 was dissolved in 2970 mg of distilled water for injection, and a hydrogel with a final concentration of 1.0 wt% was prepared. The obtained hydrogel flowed slowly when the container was inclined, and the liquid surface exhibited a tendency to move horizontally. The complex elastic modulus of the hydrogel was measured to be 15.0 ±0.14 N/m² (mean ±SD).

### Example 19

After dissolving 3000 mg of CMCNa (PM-250L, product of Dai-ichi Kogyo Seiyaku Co., Ltd., degree of substitution: 0.73) in 600 mL of water, 600 mL of tetrahydrofuran was further added. To this solution there were added 1405 mg of L-α-dioleoylphosphatidylethanolamine and 575 mg of DMT-MM as a condensation agent, and then the mixture was stirred overnight. After stirring, the tetrahydrofuran was distilled off under reduced pressure and the residue was added to ethanol for precipitation. The ethanol was removed by filtration, washing was performed again with ethanol, and the filtrate was vacuum dried to obtain a cellulose derivative, after which the phospholipid content was measured. The phospholipid content was used for calculation to determine the degree of substitution of phosphatidylethanolamine, and a value of 0.78 mol%/sugar residue was obtained.

A 30 mg portion of the obtained cellulose derivative was sterilized with ethylene oxide gas and dissolved in 5970 mg of distilled water for injection (concentration: 0.50 wt%). To 1075 mg of this solution there was added 359 mg of artificial body fluid comprising the reagents listed in the following table dissolved in 1 liter of distilled water.

| Reagent | NaCl | NaHCO₃ | KCl | K₂HPO₄ | MgCl₂6H₂O | 1M-HCl | CaCl₂ | Na₂SO₄ | (CH₂OH)₃ CNH₂ |
|---|---|---|---|---|---|---|---|---|---|
| Amount [g] | 7.996 | 0.350 | 0.224 | 0.174 | 0.305 | 40 mL | 0.278 | 0.071 | 6.057 |

The obtained hydrogel did not flow even when the container was inclined. The complex elastic modulus of the hydrogel was measured to be 23.8 ±3.3 N/m² (mean ±SD).

### Comparative Example 7

The complex elastic modulus of the hydrogel before addition of the artificial body fluid in Example 19 was measured to be 14.8 ±3.2 N/m² (mean ±SD).

### Industrial Applicability

The present invention can be utilized in industries that supply medical materials for surgeries, for example.

## Claims

1. A hydrogel comprising a polysaccharide, wherein amphiphilic side chains are bonded to the carboxyl groups of a polysaccharide with carboxyl groups on side chains, an inorganic ion other than calcium ion, and water.

2. A gel according to claim 1, wherein the inorganic ion other than calcium ion includes at least sodium ion.

3. A gel according to claim 1 or 2, wherein the polysaccharide having amphiphilic side chains bonded is a polysaccharide having phosphatidylethanolamine bonded.

4. A gel according to claim 1 or 2, wherein the amphiphilic side chains are -NH-X-CO-Y-Z.
In the formula, X is a C1-10 divalent hydrocarbon group, Y is a divalent polyalkylene oxide having oxygen atoms at both ends, and Z is a C1-24 hydrocarbon group or -CO-R¹ (R¹ being a C1-23 hydrocarbon group).

5. A gel according to any one of claims 1 to 4, which further comprises a water-soluble polymer.

6. A gel according to any one of claims 1 to 5, wherein the polysaccharide with carboxyl groups is carboxymethylcellulose.

7. A gel according to any one of claims 1 to 5, wherein the polysaccharide with carboxyl groups is hyaluronic acid.

8. A gel according to claim 3, wherein the degree of substitution of phosphatidylethanolamine is 0.3-2.0 mol%/sugar residue.

9. A kit for preparation of a hydrogel, which is constructed from two solutions: an aqueous solution of a polysaccharide, wherein amphiphilic side chains are bonded to the carboxyl groups of a polysaccharide with carboxyl groups on side chains, and an aqueous solution comprising an inorganic ion other than calcium ion.

10. A method for preparation of a hydrogel, which comprises a step of mixing an aqueous solution of a polysaccharide, wherein amphiphilic side chains are bonded to the carboxyl groups of a polysaccharide with carboxyl groups on side chains, and an aqueous solution of an inorganic ion other than calcium ion.

11. A method for preparation of a hydrogel, which comprises a step of dissolving a polysaccharide wherein amphiphilic side chains are bonded to the carboxyl groups of a polysaccharide with carboxyl groups on side chains, in an aqueous solution of an inorganic ion other than calcium ion.
